(19) Europäisches Patentamt
European Patent Office
Office européen des brevets

(11) Veröffentlichungsnummer: **0 044 448**
**A1**

(12) # EUROPÄISCHE PATENTANMELDUNG

(21) Anmeldenummer: **81105086.3**

(22) Anmeldetag: **01.07.81**

(51) Int. Cl.³: **C 07 C 69/734**
C 07 C 67/00

(30) Priorität: **04.07.80 DE 3025368**

(43) Veröffentlichungstag der Anmeldung:
**27.01.82 Patentblatt 82/4**

(84) Benannte Vertragsstaaten:
**DE FR GB IT**

(71) Anmelder: **HOECHST AKTIENGESELLSCHAFT**
**Postfach 80 03 20**
**D-6230 Frankfurt/Main 80(DE)**

(72) Erfinder: **Steglich, Wolfgang, Prof. Dr.**
**Hobsweg 77**
**D-5300 Bonn-Röttgen(DE)**

(72) Erfinder: **Schramm, Georg**
**Bergerhof 36**
**D-5190 Stolberg-Gressenich(DE)**

(72) Erfinder: **Anke, Timm, Dr.**
**Hausserstrasse 150**
**D-7400 Tübingen(DE)**

(72) Erfinder: **Oberwinkler, Franz, Prof. Dr.**
**Panoramastrasse 51**
**D-7400 Tübingen(DE)**

(54) **Verfahren zur Herstellung von Derivaten des Methylesters der 2-Methoxymethylen-3-methyl-6-phenyl-3,5-hexadiensäure.**

(57) Verfahren zur stereoselektiven Synthese von Stroliburin in der (*E*)-Konfiguration ausgehend von Zimtaldehyd und 2-Oxo-buttersäure.

**EP 0 044 448 A1**

Verfahren zur Synthese von Strobilurin

Die Erfindung betrifft ein Verfahren zur Herstellung von Strobilurin auf synthetischem Wege.

Kürzlich wurden aus Kulturen von Strobilurus tenacellus die Antibiotika Strobilurin A und B isoliert, die starke antifungische Eigenschaften besitzen und auch gegen humanpathogene Pilze wirksam sind (vgl. T. Anke, F. Oberwinkler, W. Steglich und G. Schramm, J. Antibiot. 30, 806 (1977) und G. Schramm, W. Steglich, T. Anke und F. Oberwinkler, Chem. Ber. 111, 2779 (1978)). Es handelt sich um 1-Arylhexatriene der Struktur 1 (Strobilurin A) bzw. 2 (Strobilurin B), die an der terminalen Doppelbindung eine für die biologische Wirkung entscheidene ß-Methoxyacrylat-Gruppierung in der (E)-Konfiguration besitzen.

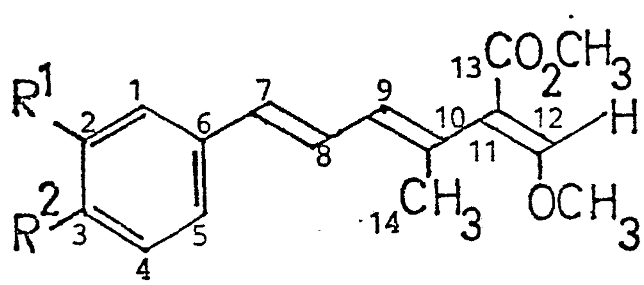

1, $R^1 = R^2 = H$
2, $R^1 = OCH_3$, $R^2 = Cl$

Da nur Verbindungen der angegebenen (E)-Konfiguration eine gute Hemmwirkung besitzen, bestand die Aufgabe darin, eine stereoselektive Synthese zu entwickeln.

Es wurde nun gefunden, daß eine stereoselektive Synthese von Strobilurin nach dem nachfolgend beschriebenen Verfahren möglich ist.

Die Erfindung betrifft daher ein Verfahren zur Herstellung von synthetischem Strobilurin in der (E)-Konfiguration,

0044448

das dadurch gekennzeichnet ist, daß man einen entsprechend substituierten Zimtaldehyd mit 2-Oxobuttersäure in methanolischer KOH zum 3(Z),5(E)-3-Methyl-2-oxo-6-phenyl-3,5-hexadiensäure-Kaliumsalz kondensiert, das Kaliumsalz mit Methanol* in den entsprechenden Methylester überführt, das Reaktionsprodukt mit Methoxymethylentriphenylphosphoran zum 9(Z)-Strobilurin umsetzt und das Rohprodukt nach chromatographischer Reinigung an Kieselgel mit einer Quecksilberdampflampe in Aceton unter Zusatz von etwas Benzol bestrahlt.

*/Thionylchlorid

Das erfindungsgemäße Verfahren eignet sich insbesondere zur Herstellung von Strobilurin A. Die Reaktion verläuft nach folgendem Schema:

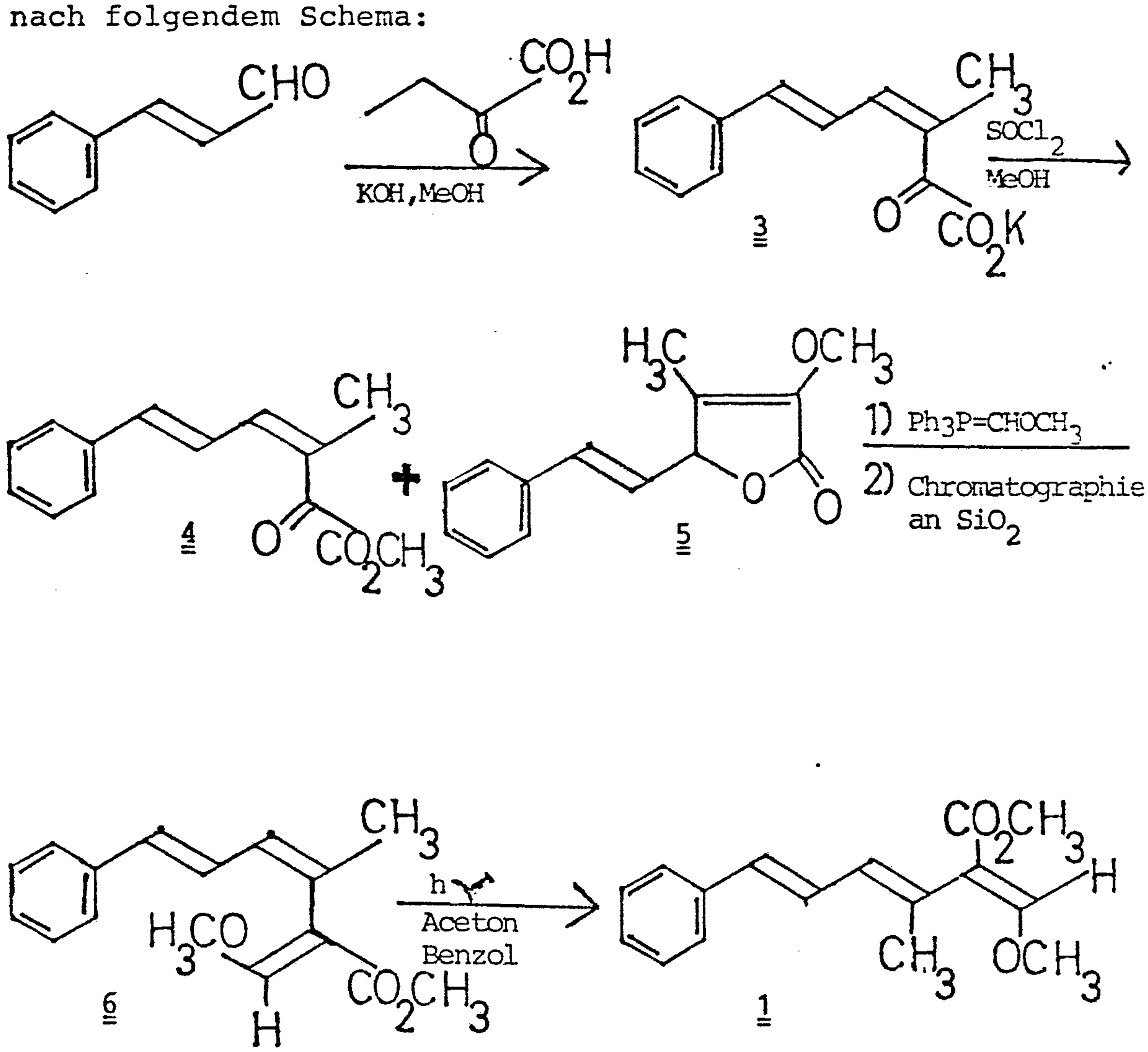


3

4

5

6

1

Bei der Synthese des Strobilurins A (1) geht man von Zimtaldehyd und 2-Oxobuttersäure aus, die in methanolischer KOH zum 3(Z),5(E)-3-Methyl-2-oxo-6-phenyl-3,5-hexadiensäure-Kaliumsalz (3) kondensiert werden (vgl. E.D. Stecher und H.F. Ryder, J. Am. Chem. Soc. 74, 4392 (1952)). Die Veresterun g des Kaliumsalzes erfolgt zweckmäßig mit $SOCl_2$/Methanol, entsprechende den Angaben S. Guttmann und R. Boissonas, Helv. Chim. Acta 41, 1852 (1958). Der Methylester 4 wird noch von ca 15-18 % des isomeren Lactons 5 begleitet. Das Gemis kann ohne weitere Reinigung mit Methoxymethylentriphe.lphosphoran entsprechend den Angaben in der Literatur (G. Wittig und E. Knauss, Angew. Chem. 71, 127 (1959); S.G. Levine, J. Am. Chem. Soc. 80, 6150 (1958); G. Wittig , W. Böll und K.-H. Krück, Chem. Ber. 95, 2514 (1962); G. Wittig und M. Schlosser, Chem. Ber. 94, 1373 (1961)) zum kristallinen 9(Z)-Strobilurin (6) umgesetzt werden. Das Rohprodukt enthält etwas 9(Z),11(Z)-Strobilurin A, das bei der chromatographischen Reinigung an Kieselgel in das thermodynamisch stabilere Isomere 6 übergeht. Bestrahlen mit einer Quecksilberdampflampe (Solidexfilter) in Aceton unter Zusatz von etwas Benzol führt zur Konfigurationsumkehr an $\Delta^9$ unter Bildung von Strobilurin A (1), das sich in allen spektroskopischen Eigenschaften und der biologischen Wirkung mit dem Naturstoff als identisch erweist.

Die Wittigreaktion mit Methoxymethylen-triphenylphosphoran wurde bisher noch nicht auf 2-Oxocarbonsäureester angewandt. Die vollständige photochemische Isomerisierung von 6 zum all-*trans*-Derivat 1 ist überraschend.

Ausgehend von im aromatischen Ring substituierten Zimtaldehyden sind auch Strobilurin B (2) und Analoga auf dem beschriebenen Weg zugänglich.

Versuche:

3(Z),5(E)-Methyl-2-oxo-6-phenyl-3,5-hexadiensäure-Kaliumsalz (3)

Zu einem auf 10°C gekühlten Gemisch von 10.2 g 2-Oxobuttersäure und 13.2 g Zimtaldehyd werden unter $N_2$ als Schutzgas 8.4 g KOH in 25 ml Methanol so zugetropft, daß die Temperatur auf 25°C ansteigt und bei weiterer Zugabe konstant bleibt. Beim Zutropfen des letzten Viertels der Lauge erreicht man durch Erhöhung der Tropfgeschwindigkeit einen Temperaturanstieg auf 45°C. Sofort nach beendigter Zugabe wird das Reaktionsgemisch rot, und das Produkt fällt aus. Man rührt noch 1 h bei 25°C und läßt das Gemisch über Nacht im Eisschrank stehen. Danach wird abgesaugt und das Produkt 2-3mal mit wenig trockenem Methanol und anschließend 3-5mal mit trockenem Ether gewaschen. Das Kaliumsalz ist danach hellgelb und feinpulvrig. Ausbeute: 13.5 g (51.2 %); Schmp. 210-215°C (Zers.).

3(Z),5(E)-3-Methyl-2-oxo-6-phenyl-3,5-hexadiensäure-methylester (4)

Zu 84 ml trockenem Methanol werden bei -5°C 4.58 g Thionylchlorid getropft. Danach gibt man 9.0 g (35 mmol) 3-Methyl-2-oxo-6-phenyl-3,5-hexadiensäure-Kaliumsalz (3) zu, läßt auf Raumtemperatur kommen und erhitzt anschließend 20 min unter Rückfluß. Nach DC-Kontrolle auf vollständige Veresterung wird KCl abgesaugt, das Filtrat eingeengt und nachgefallenes Salz durch eine G3-Fritte entfernt. Eindampfen der Mutterlauge und Trocknen im Ölpumpenvakuum gibt 8.0 g eines Öls, das nach einigen Tagen im Kühlschrank wachsartig erstarrt. Das so erhaltene Rohprodukt enthält nach dem $^1$H-NMR-Spektrum ca. 15-18 % 4-Methyl-3-methoxy-5-styryl-2,5-dihydrofuran-2-on (5). Es kann ohne weitere Reinigung für den nächsten Reaktionsschritt eingesetzt werden.

Chromatographie an Kieselgel (Eluent: Methanol) ergibt den reinen Ester 4; Schmp. 49-50°C.

$^1$H-NMR([$D_6$] Aceton): 2.04 (d, J=1.2 Hz, C-$CH_3$); 3.92 (s, $OCH_3$); 7.18 (d, J= 15.6 Hz, 6-H); 7.24 (dq, J= 9.4 + 1.2 Hz, 4-H); 7.4 (dd, verdeckt, 5-H); 7.5 (m, 3H); 7.7 (m, 2H).

9(Z)-Strobilurin A (6)

6.5 g (21 mmol) Methoxymethyl-triphenylphosphoniumchlorid werden in 90 ml absolutem Ether suspendiert und unter $N_2$ mit 2.1 g (19 mmol) Kalium-tert-butylat versetzt. Es wird 35 min bei Raumtemperatur gerührt und dann 3.2 g (14 mmol) roher 3(Z),5(E)-3-Methyl-2-oxo-6-phenyl-3,5-hexadiensäuremethylester (4) in 20 ml Ether sehr schnell zugetropft, wobei sich die tiefrote Lösung nahezu entfärbt. Nach weiteren 15 min Rühren gießt man das Gemisch in 100 ml wäßrige Ammoniumchloridlösung, schüttelt aus, trennt den Ether ab und extrahiert noch zweimal mit Ether, Eindampfen der über $MgSO_4$ getrockneten organischen Phasen und Aufgeben des in wenig Essigester gelösten Rückstandes auf eines mit Petrolether (40-60°C) angesetzte Kieselgelsäule (Mallinckrodt) liefert nach Elution mit Petrolether und anschließend Petrolether/Toluol (1:1) relativ reines 9(Z)-Strobilurin A (6), das nach dem Eindampfen kristallisiert. Ausbeute 1.45 g (40 %, bezogen auf den rohen Ketoester 4); blaßgelbliche Kristalle vom Schmp. 75-77°C.

$^1$H-NMR ([$D_6$] -Aceton): 1.96 (d, J= 1.0 Hz, C-$CH_3$); 3.65 (s, $OCH_3$); 3.88 (s, $CO_2CH_3$); 6.15 (dq, J= 11.0 + 1.0 Hz, 9-H); 6.55 (d, J= 15.6 Hz, 7-H), 7.22 (dd, J= 15.6 + 11.0 Hz, 8-H); 7.38 (s, 12-H); ~7.4 (m, 5H).

Strobilurin A (1)

0.30 g 9(Z)-Strobilurin A (6) werden in 6 ml Aceton und 0.6 ml Benzol 1 h mit einer Quecksilberdampflampe mit

Solidexfilter im Bestrahlungskarussell (Fa. Dema, Bornheim) bestrahlt. Danach ist nach dem DC die Isomerisierung vollständig abgelaufen [$R_f$-Werte: 0.47 (4), 0.50 (5); Hexan/$CCl_4$/$CHCl_3$/EtOAc = 4/3/1/1 auf Kieselgelfertigplatten Merck ]. Man rotiert ein und chromatographiert an ® Sephadex LH 20 mit Methanol. Ausbeute: 0.255 g (85 %), farbloses Öl; identisch mit dem Naturprodukt.

$^1$H-NMR ([$D_6$]-Aceton): 1.92 (d, *J*= 1.3 Hz, C-$CH_3$); 3.67 (s, $OCH_3$); 3.90 (s, $CO_2CH_3$); 6.19 (dq, *J*= 10.0 + 1.3 Hz, 9-H); 6.48 (d, *J*= 15.6 Hz, 7-H); 6.75 (dd, *J*= 15.6 + 10.0 Hz, 8-H); 7.48 (s, 12-H); ~7.4 (m, 5H).

Patentanspruch:

Verfahren zur Herstellung von synthetischem Strobilurin in der (E)-Konfiguration, dadurch gekennzeichnet, daß man einen entsprechend substituierten Zimtaldehyd mit 2-Oxobuttersäure in methanolischer KOH zum entsprechend substituierten 3(Z),5(E)-3-Methyl-2-oxo-6-phenyl-3,5-hexadiensäure-Kaliumsalz kondensiert, das Kaliumsalz mit Methanol in den entsprechenden Methylester überführt, das Reaktionsprodukt mit Methoxymethylentriphenylphosphoran zum 9(Z)-Strobilurin umsetzt und das Rohprodukt nach chromatographischer Reinigung an Kieselgel mit einer Quecksilberdampflampe in Aceton unter Zusatz von etwas Benzol bestrahlt.

0044448

Europäisches Patentamt

# EUROPÄISCHER RECHERCHENBERICHT

Nummer der Anmeldung

EP 81105086.3

| EINSCHLÄGIGE DOKUMENTE | | | KLASSIFIKATION DER ANMELDUNG (Int Cl ) |
|---|---|---|---|
| Kategorie | Kennzeichnung des Dokuments mit Angabe, soweit erforderlich, der maßgeblichen Teile | betrifft Anspruch | |
| | --------------------------------<br>Keine Entgegenhaltungen. | | C 07 C 69/734<br>C 07 C 67/00 |
| | | | RECHERCHIERTE SACHGEBIETE (Int Cl ) |
| | | | C 07 C 69/00<br>C 07 C 67/00 |
| | | | KATEGORIE DER GENANNTEN DOKUMENTE<br>X: von besonderer Bedeutung<br>A: technologischer Hintergrund<br>O: nichtschriftliche Offenbarung<br>P: Zwischenliteratur<br>T: der Erfindung zugrunde liegende Theorien oder Grundsätze<br>E: kollidierende Anmeldung<br>D: in der Anmeldung angeführtes Dokument<br>L: aus andern Gründen angeführtes Dokument |
| X | Der vorliegende Recherchenbericht wurde für alle Patentansprüche erstellt. | | &: Mitglied der gleichen Patentfamilie, übereinstimmendes Dokument |

| Recherchenort | Abschlußdatum der Recherche | Prüfer |
|---|---|---|
| WIEN | 13-08-1981 | HOFBAUER |

EPA form 1503.1 06.78